# EUROPEAN PATENT SPECIFICATION

(11) **EP 3 865 503 B1**
(45) Date of publication and mention of the grant of the patent: **28.01.2026**
(21) Application number: 19871764.7
(22) Date of filing: 08.10.2019
(51) Int. Cl.: C07K 14/46, G01N 33/50, G01N 33/68

(54) **LEUCINE RICH ALPHA-2 GLYCOPROTEIN COMPOSITION**
LEUCINREICHE ALPHA-2-GLYKOPROTEINZUSAMMENSETZUNG
COMPOSITION DE GLYCOPROTÉINE ALPHA-2 RICHE EN LEUCINE

(30) Priority: 09.10.2018 JP 2018190904
(43) Date of publication of application: 18.08.2021
(73) Proprietor: Sekisui Medical Co., Ltd., Tokyo 103-0027 (JP); National Institutes of Biomedical Innovation, Health and Nutrition, Ibaraki-shi, Osaka 567-0085 (JP)
(72) Inventor: TAKAYAMA Shigeo, Tokyo 103-0027 (JP); NAKA Tetsuji, Ibaraki-shi, Osaka 567-0085 (JP); SERADA Satoshi, Ibaraki-shi, Osaka 567-0085 (JP)
(74) Representative: Hoffmann Eitle
(86) International application number: PCT/JP2019/039565
(87) International publication number: WO 2020/075692

(56) References cited:
- WO-A1-2011/125877
- JP-A- 2011 116 697
- JP-A- 2012 092 095
- JP-A- 2016 079 170
- RAMIL CODINA ET AL: "Cytochrome c-induced lymphocyte death from the outside in: inhibition by serum leucine-rich alpha-2-glycoprotein-1", APOPTOSIS ; AN INTERNATIONAL JOURNAL ON PROGRAMMED CELL DEATH, KLUWER ACADEMIC PUBLISHERS, BO, vol. 15, no. 2, 23 October 2009 (2009-10-23), pages 139 - 152, XP019766422, ISSN: 1573-675X
- RESEARCH ATHENS ET AL: "6-�16-�121807-� Leucine-�rich Alpha 2 Glycoprotein 1, Human Plasma SDS -SAFETY DATA SHEET 1. IDENTIFICATION PRODUCT NAME Leucine-�rich Alpha 2 Glycoprotein 1, Human Plasma (LRG1) PRODUCT NO. 16-�16-�121807 BRAND Athens Research and Technology Use of substance", 14 November 2013 (2013-11-14), pages 1 - 6, XP055925397, Retrieved from the Internet <URL:https://www.athensresearch.com/images/_dynamic/Leucine-rich-Alpha-2-Glycoprotein-1-Human-Plasma-16-16-121807-SDS-Sheet-Revised-14Nov13.pdf> [retrieved on 20220526]
- IBL: "Instruction for Use Code No Mouse LRG Assay Kit -IBL 96 Well", 1 July 2015 (2015-07-01), pages 1 - 2, XP055925398, Retrieved from the Internet <URL:https://www.ibl-america.com/content/elisa/27785.pdf> [retrieved on 20220526]
- SERADA SATOSHI, FUJIMOTO MINORU, OGATA ATSUSHI, TERABE FUMITAKA, HIRANO TORU, IIJIMA HIDEKI, SHINZAKI SHINICHIRO, NISHIKAWA TEPPEI: "iTRAQ-based proteomic identification of leucine-rich a -2 glycoprotein as a novel inflammatory biomarker in autoimmune diseases", ANNALS OF THE RHEUMATIC DISEASES, vol. 69, no. 4, 2010, pages 770 - 774, XP009527213, DOI: 10.1136/ard.2009.118919

## Description

### TECHNICAL FIELD

The present invention relates to a leucine-rich α2 glycoprotein composition. More specifically, the invention relates to a composition with which leucine-rich α2 glycoprotein can be preserved stably for a long time.

### BACKGROUND ART

Leucine-rich α2 glycoprotein (hereinafter, sometimes referred to as LRG) is one of serum proteins. It is reported that LRG is a glycoprotein of about 50 kDa and is secreted from neutrophils . J Leukoc Biol. 2002 72(3) : 478-85.2002

Recently, the relevance of LRG in a biclogical sample to specific diseases has been studied. For example, JP-A-2010-286279 reports that detecting LRG in a biological sample is useful for testing autoimmune diseases such as Behcet's disease.

To quantitatively determine the component to be measured in a biological sample, it is necessary to use a calibration sample. The calibration sample is a sample containing the component to be measured and is used as an internal standard, a concentration calibration standard (calibrator) or the like. To obtain an accurate quantitative value, a calibration sample which is stable in spite of the passage of time or the temperature is required. That is, it is believed that, when the component to be measured in the calibration sample is decomposed and/or denatured during the preservation, the measured value is also affected. For quantitative determination of LRG, a method using the enzyme-linked immunosorbent assay (ELISA) or the like has been known so far, but the calibration sample has not been examined yet.

Ramil Codina *et al.* (RAMIL CODINA ET AL, APOPTOSIS; AN INTERNATIONAL JOURNAL ON PROGRAMMED CELL DEATH, KLUWER ACADEMIC PUBLISHERS, BO, vol. 15, no. 2, 23 October 2009 (2009-10-23), pages 139-152) relates to c-induced lymphocyte death from the outside in: inhibition by serum leucine-rich alpha-2-glycoprotein-1.

Research Athens et al.: ("6-16-121807- Leucine-rich Alpha 2 Glycoprotein 1, Human Plasma SDS -SAFETY DATA SHEET 1. IDENTIFICATION PRODUCT NAME Leucine-rich Alpha 2 Glycoprotein 1, Human Plasma (LRG1) PRODUCT NO. 16-16-121807 BRAND Athens Research and Technology Use of substance",14 November 2013 (2013-11 -14), pages 1 -6, XP055925397,Retrieved from the Internet:URL:https://www.athensresearch.com/images/_dynamic/Leucine- rich-Alpha-2-Glycoprotein-1 -Human-Plasma-16-16-121807-SDS- Sheet-Revised-14Nov13.pdf) relates to an LRG1 detection kit.

Mousse LRG Assay kit ("Instruction for Use Code No Mouse LRG Assay Kit -IBL 96 Well", 1 July 2015 (2015-07-01), pages 1-2, XP055925398, Retrieved from the Internet:URL: https://www.ibl-ameri ca.com/content/elisa/27785.pdf) relates to a detection kit for Mouse LRG.

JP 2011 116697 relates to a neovascularization-inducing molecule.

### SUMMARY OF INVENTION

### TECHNICAL PROBLEM

The present inventors have tried to produce a calibration sample solution containing LRG. As a result, it was found that, when LRG is added to a solution having a pH of less than 7, the stability of LRG is poor, and LRG is gradually decomposed and lost with the time.

An object of the invention is to provide a leucine-rich α2 glycoprotein composition which contains leucine-rich α2 glycoprotein and which has high preservation stability.

### SOLUTION TO PROBLEM

As a result of further investigation, the inventors have found that the stability of LRG improves when the pH of a solution containing LRG is kept in a specific range. It was also found that the stability of LRG further improves when the solution has a salt concentration in a specific range. The invention is based on the findings.

The invention concerns a leucine-rich α2 glycoprotein composition comprising leucine-rich α2 glycoprotein,
wherein a pH of the composition is 7.8 to 9.0,
wherein a concentration of a salt of the composition is 300 mM to 600 mM,
wherein the salt is a sodium salt, potassium salt or magnesium salt, and
wherein the concentration of the leucine-rich α2 glycoprotein in the composition is 0.1 to 1000 µg/mL.

Further aspects of the invention are defined in the claims.

### BRIEF DESCRIPTION OF DRAWINGS

[Fig. 1] A graph showing the changes in LRG amount of solutions having various pH values with time, where the initial values are regarded as 100%.
[Fig. 2] A graph showing the changes in LRG amount of solutions having various salt concentrations with time, where the initial values are regarded as 100%.
[Fig. 3] A graph showing the changes in LRG amount of solutions having various pH values with time, where the initial values are regarded as 100%.

### DESCRIPTION OF EMBODIMENTS

### (Leucine-Rich α2 Glycoprotein)

Leucine -rich α2 glycoprotein or LRG is a glycoprotein of about 50 kDa. LRG is one of serum proteins, and it is said that about 3.0 µg/mL is cont ained in the serum of a healthy individual. It is reported that LRG is secreted from neutrophils. The relevance of a change in the LRG amount in the body to various diseases, for example, to tuberculosis or a tumor, has been studied recently, and it is required to accurately me asure the amount of LRG in the living body to accur ately di agnose these diseases.

LRG can be me asured using a known method such as an immunologic al method. The immunologic al methods include ELISA, enzyme immunoassay, surface plasmon resonance, latex agglutination immuno ass ay (LTIA), chemiluminescence immuno ass ay, electrochemiluminescence immuno ass ay, fluorescent antibody technique, r adioimmuno ass ay, immunoprecipitation, Western blotting, immunochromatography, high performance liquid chromatogr aphy (HPLC) and the like.

As the LRG contained in the composition of the invention, commerci ally avail able LRG may be used, and LRG which has been produced or purified by a user may also be used. As the LRG cont ained in the composition of the invention, LRG which has been produced *in vitro* may be used, and LRG which has been extracted from a living body may also be used.

### (pH)

Considering the stability of LRG, the pH of the leucine -rich α2 glycoprotein composition of the invention is 7. 8 to 9.0, most prefer ably 8. 0 to 9. 0.

The pH c an be adj usted using a pH adj usting re agent known to one skilled in the art such as sodium hydroxide.

### (Salt Concentration)

The salt concentration of the LRG composition of the invention is 300 mM to 600 mM. By keeping the salt concentration within this range, the stability of LRG c an be further improved.

As the kind of the salt cont ained in the LRG composition of the invention, sodium s alts, pot assiums alts and magnesium salts an be used. A sodium salt is more prefer ably used. To supply a sodium s alt, sodium chloride is most prefer ably used. More than one kind of salt c an also be used in combination.

### (Leucine-Rich α2 Glycoprotein Composition)

In the present specification, the leucine -rich α2 glycoprotein composition (herein after, sometimes referred to as the LRG composition) means a solution cont aining LRG which is used for measuring LRG. The LRG composition of the invention c an be suit ably used as ac alibr ation s ample solution for LRG measurement. In the specification, the calibration s ample solution means a s ample solution cont aining the substance to be me asured at a certain concentration which is used for accurately me asuring the substance to be me asured, and a st and ard substance, a calibrator, a control, an intern al standard substance and the like are the calibration sample solutions. A form for supplying the LRG composition of the invention is a form in a solution state prepared in advance by mixing LRG and a solvent having a pH of 7.8 to 9.0.Another form for supplying the LRG composition of the invention is a form in which LRG and the solvent are prepared separately and in which the two are mixed into a solution state before the use.

The concentration of LRG in the LRG composition of the is invention 0.1 to 1000 µg/mL.

In this regard, "using the leucine -rich α2 glycoprotein composition" in the method for me asuring leucine -rich α2 glycoprotein means that the leucine -rich α2 glycoprotein composition is used to accurately me asure leucine -rich α2 glycoprotein and means that the leucine -rich α2 glycoprotein composition is used as a calibration sample solution (a standard substance, a calibrator, a control, an intern al standard substance and the like) for example.

The composition of the LRG composition of the invention is defined in the claims When LRG is me asured by an immunologic al measurement method, the effects of the invention should not be imp aired, and the whole or apart of the re action constituting the measurement system such as antigen -antibody re action, labeling re action for detection with biotin / avidin and enzymatic reaction should not be interrupted. A component which is gener ally used in an immunologic al me asurement method, for ex ample, a buffer such as acetic acid, citric acid, phosphoric acid, HEPES, MES, Tris, glycine, boric acid, carbonic acid and a Good' s buffer, a component for inhibiting non -specific re action (a nonionic surf act ant such as Tween20 and TritonX-100 or the like), a component which promotes antigen -antibody reaction (a polymer such as polyethylene glycol, polyvinylpyrrolidone and aphospholipid polymer or the like), a glycoprotein other than LRG or a peptide (albumin, casein or the like), an amino acid, a sacch aride (sucrose, cyclodextrin or the like), a preservative (sodium azide, ProClin950 or the like) or the like c an be appropriately selected and used depending on the purpose.

The LRG composition of the invention c an be preserved in a known cont ainer. The material of the preservation cont ainer that c an be used for the LRG composition of the invention can be polypropylene, polystyrene or glass although the material is not limited to these materials. The form of the preservation cont ainer may be either a hard type or a soft type, and examples include an ampule, a vial, a soft bag, an injection -type cont ainer and the like.

LRG c an be produced by LRG-expressing cells. The LRG-expressing cells c an be obtained by transformation of a host with an expression vector cont aining DNA encoding LRG. The host cells can be microorganism cells, insect cells, mammalian cells or the like, which are prokaryotes or euk aryotes. As the mammalian cells, for example, HepG2 cells, HEK293 cells, HeLa cells, human FL cells, monkey COS -7 cells, monkey Vero cells, Chinese hamster ovary cells (abbreviated to CHO cells below), dhfr gene -knockout CHO cells, mouse L cells, mouse AtT -20 cells, mouse myeloma cells, rat H4IIE -C3 cells, rat GH3 cells and the like c an be used.

A plasmid obtained in the above manner c an be introduced into the host cells by a gener al genetic engineering method. The tr ansformant c an be cultured by a gener al method used for culturing microorganisms or culturing insect cells or mammali an cells. The LRG protein c an be obtained by a combination of methods which are gener ally used for isolation/purific ation of gener al proteins.

In the specification, "preservation stabilizing" or that "the stability improves" means that, because most of LRG cont ained in a solution cont aining LRG is maintained without being decomposed or without a structural change for along time, the initial LRG value of the solution and the measured value after preservation do not differ greatly. More specific ally, for ex ample, it means that, because 75% or more of LRG cont ained in a solution cont aining LRG is maintained without being decomposed or without a structural change for 29 days, the measured LRG value of the solution after preservation at 37°C for 29 days is 75% or more of the initial value. The LRG composition of the invention has a measured LRG value after preservation at 37°C for 29 days of, based on the initial amount, prefer ably 80% or more, more prefer ably 90% or more, further prefer ably 95% or more, most prefer ably 96% or more. The LRG composition of the invention has long -term stability also at a high temper ature of 37°C, but an LRG composition which is preserved at a low temper ature or at normal temper ature is not excluded from the scope of the invention.

### (Biologic al S ample for LRG Me asurement)

The biologic al s ample for the me asurement of LRG is not particularly limited as long as LRG can be me asured but is blood, serum, plasma, cerebral spinal fluid (CSF), urine, stool or the like. Serum or plasma is prefer ably used, and serum is further prefer ably used. The living body or the subject is not particularly limited as long as the living body or the subject is a mammal and includes a human or an animal (for ex ample, a monkey, a dog, a c at, a mouse, a guine a pig, a r at, a hamster, a horse, a cow and a pig), and a human is prefer able. The biologic al s ample may be appropri ately subjected to pretreatment when necess ary.

### (Measurement Kit for Leucine -Rich α2 Glycoprotein)

With the kit for me asuring leucine -rich α2 glycoprotein (herein after, sometimes referred to as an LRG measurement kit) of the invention, LRG c an be me asured accurately using an LRG composition. An example of the LRG measurement kit is a kit using an immunologic al method. The LRG measurement kit of the invention can include a reagent for measuring the LRG concentration of a human body by an immunologic al method. The immunologic al methods include ELISA, enzyme immuno ass ay, surf ace plasmon reson ance, latex agglutination immuno ass ay (LTIA), chemiluminescence immuno ass ay, electrochemiluminescence immuno ass ay, fluorescent antibody technique, radioimmunoassay, immunoprecipitation, Western blotting, immunochromatography, high-performance liquid chromatogr aphy (HPLC) and the like. The immunologic al method is prefer ably latex agglutination immuno ass ay (LTIA).

The LRG me asurement kit of the invention can be used for di agnosing autoimmune dise ases, tuberculosis, tumors, inflammatory diseases and inflammatory bowel diseases (ulcer ative colitis and Crohn' s disease).

The LRG me asurement kit of the invention c an also include a user guide or the like. The kit may also include any constituent element such as a buffer, a stabilizing agent, a specimen diluent, a pH adjustment agent and a reaction cont ainer.

### (Preservation Stabilizing Method for LRG Composition)

The preservation stabilizing method for the LRG composition of the invention includes a step of bringing leucine -rich α2 glycoprotein into contact with a solvent having a pH of 7.8 to 9.0. The solvent is not particularly **7.8** to 9.0 limited as long as the pH is 7.8 to 9.0. The solvent c an contain a buffer such as HEPES, MES, CHES and Tris, and the concentration of the buffer can be 1 to 1000 mM. "Bringing into contact" includes adjusting the pH of an LRG-containing solution having a pH that is not 7.8 to 9.0 to 7.8 to 9.0 in addition to adding LRG to the solvent having a pH of 7.8 to 9.0.

Next, the invention is explained specific ally referring to Examples, but the Examples do not limit the scope of the invention. In the specification, % indicates weight % unless otherwise explained.

### EXAMPLES

### [Example 1] Examin ation 1 of Changes in LRG Amount of Solutions Having Various pH Values with Time

After producing LRG standard antigen solutions having various pH values, a preservation test was conducted, and the changes in LRG amount of the LRG standard antigen solutions with time were examined. The influence of the pH on the preservation stability of LRG was thus examined. The LRG used was produced using CHO (Chinese hamster cells). Specific ally, the LRG was prepared by introducing human LRG gene to CHO -K1 cells using a plasmid, culturing the LRG gene -introduced CHO -K1 cells in a serum-free medium and recovering the human LRG recombin ant protein from the culture supernat ant.

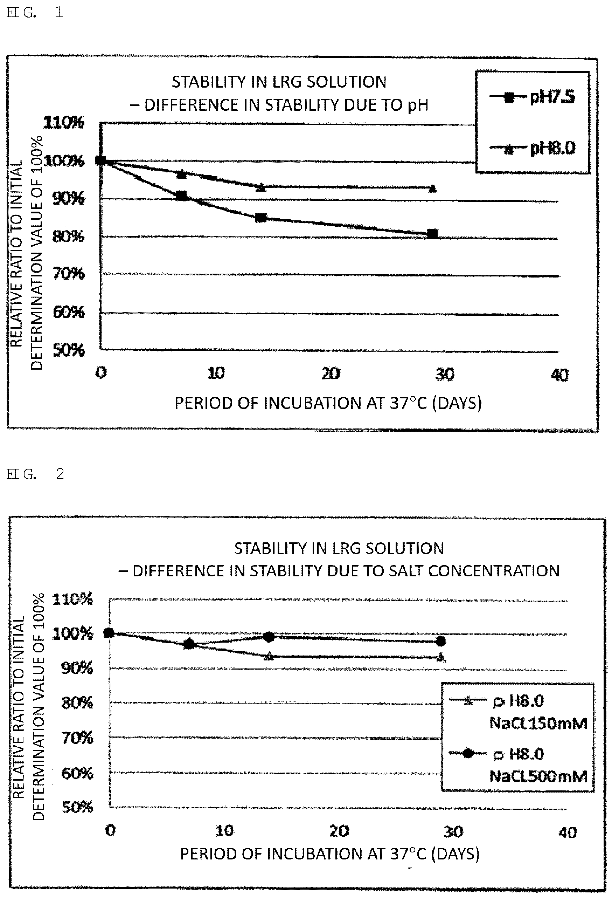

LRG standard antigen solutions having the composition in Table 1 below were produced, and the pH values were controlled to 7.5 or 8. 0 using a 4N sodium hydroxide solution. The LRG standard antigen solutions were stored at 37°C for 29 days, and the changes in LRG amount with time were observed. The sample composition and the experiment al results are shown in T ables 1 and 2 and Fig. 1.

**[Table 1]**

| Component | Concentration |
|---|---|
| Bovine Serum Albumin (BSA PF) | 1% |
| HEPES | 50 mM |
| Sodium Chloride | 150 mM |
| TWEEN 20 | 0. 01% |
| E DIA 3Na | 5 mM |
| ProClin950 | 0.05% |
| Purified Water | Necess ary Amount |
| LRG | 10.0 µg/mL |

**[Table 2]**

| S ample No. | PH of LRG Standard Antigen Solution | Measured Value After 29 Days (Initial Measured Value is 100%) |
|---|---|---|
| 1 | 7. 5 | 81% |
| 2 | 8. 0 | 93% |

The me asured value of the LRG standard antigen solution having a pH adjusted to 8. 0 after preservation at 37°C for 29 days was 93% of the initial LRG amount. The measured value of the LRG st and ard antigen solution having a pH adj usted to 7.5 was 81% of the initi al LRG amount. Therefore, it was shown that the preservation stability of LRG is excellent when the pH of the LRG standard antigen solution is 8. 0 rather than 7. 5.

### [Example 2] Examination of Changes in LRG Amount of Solutions Having Various Salt Concentrations with Time

The concentration of sodium chloride was changed from 150 mM to 500 mM in s ample No. 2 produced in Example 1, and a preservation test was conducted. The influence of the salt concentration on the preservation stability of LRG was thus examined. The sample compositions and the experimental results are shown in Table 3 and Fig. 2.

**[Table 3]**

| S ample No. | Salt Concentration (mM) | Measured Value After 29 Days (Initial Measured Value is 100%) |
|---|---|---|
| 2 | 150 | 93% |
| 3 | 500 | 98% |

It was shown that the preservation stability of LRG improves with the LRG standard antigen solution having an increased salt concentration of 500 mM compared to the LRG standard antigen solution having a salt concentration of 150 mM. While the measured value of the LRG standard antigen solution having a salt concentration of 150 mM after preservation at 37°C for 29 days was 93% of the initial LRG amount, the me asured value of the LRG standard antigen solution having a salt concentration of 500 mM after preservation at 37°C for 29 days was 98% of the initi al LRG amount.

### [Example 3] Examin ation 2 of Changes in LRG Amount of Solutions Having Various pH Values with Time

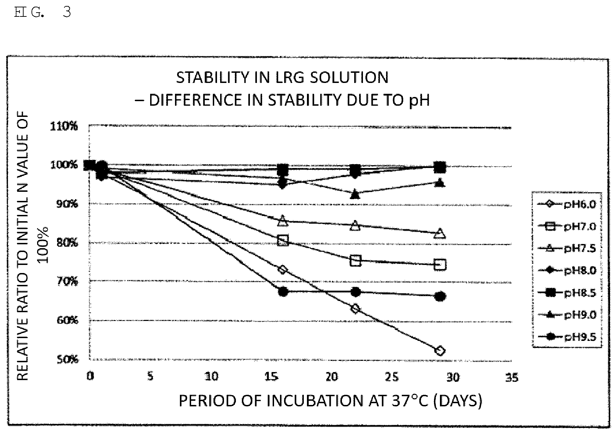

LRG standard antigen solutions having the same composition as that of the samples prepared in Example 1 were produced except that the buffers shown in Table 4 below were used and that the pH was adjusted to 6.0, 7.0, 7.5, 8.0, 8.5, 9.0 or 9.5. The LRG standard antigen solutions were stored at 37°C for 29 days, and the changes in LRG amount with time were observed. The sample compositions and the experiment al results are shown in Table 4 and Fig. 3.

**[Table 4]**

| Sample No. | Buffer | pH | Measured Value After 29 Days (Initial Measured Value is 100%) |
|---|---|---|---|
| 4 | MOPS | 6. 0 | 52% |
| 5 | HEPES | 7.0 | 75% |
| 6 | HEPES | 7. 5 | 83% |
| 7 | HEPES | 8. 0 | 100% |
| 8 | HEPES | 8. 5 | 100% |
| 9 | CHES | 9. 0 | 96% |
| 10 | CHES | 9. 5 | 67% |

From the above results, it was shown that the preservation stability of LRG improves when the pH of the LRG standard antigen solution is adjusted to 7.0 to 9.3.

### INDUSTRIAL APPLICABILITY

The leucine-rich α2 glycoprotein composition of the invention has excellent preservation stability. Thus, when the leucine -rich α2 glycoprotein composition of the invention is used as a c alibr ation s ample, LRG in a biologic al sample c an be quantitatively measured accurately, and disease can accurately be di agnosed.

## Claims

1. A leucine-rich α2 glycoprotein composition comprising leucine-rich α2 glycoprotein, wherein a pH of the composition is 7.8 to 9.0,
wherein a concentration of a salt of the composition is 300 mM to 600 mM,
wherein the salt is a sodium salt, potassium salt or magnesium salt, and
wherein the concentration of the leucine-rich α2 glycoprotein in the composition is 0.1 to 1000 µg/mL.

2. The leucine-rich α2 glycoprotein composition according to claim 1, wherein the salt is a sodium salt, optionally wherein the salt is sodium chloride.

3. The leucine-rich α2 glycoprotein composition according to claim 1 or 2, wherein the pH of the composition is 8.0 to 9.0.

4. The leucine-rich α2 glycoprotein composition according to any one of claims 1 to 3, wherein the α2 glycoprotein composition is filled in a preservation container.

5. Use of the leucine-rich α2 glycoprotein composition according to any one of claims 1 to 4 as a calibration sample solution for measuring the concentration of leucine -rich α2 glycoprotein.

6. A method for measuring the concentration of leucine-rich α2 glycoprotein comprising: using the leucine-rich α2 glycoprotein composition according to any one of claims 1 to 4 as a calibration sample solution.

7. A kit comprising the leucine-rich α2 glycoprotein composition according to any one of claims 1 to 4.

8. A preservation stabilizing method for leucine-rich α2 glycoprotein comprising:
bringing leucine-rich α2 glycoprotein into contact with a solvent having a pH of 7.8 to 9.0 wherein a concentration of a salt in the solvent is 300 mM to 600 mM,
wherein the salt is a sodium salt, potassium salt or magnesium salt, and
wherein the concentration of the leucine-rich α2 glycoprotein is 0.1 to 1000 µg/mL.

9. The preservation stabilizing method for leucine-rich α2 glycoprotein according to claim 8, wherein the salt is a sodium salt, optionally wherein the salt is sodium chloride.

10. The preservation stabilizing method for leucine-rich α2 glycoprotein according to claim 8 or 9, wherein the pH of the composition is 8.0 to 9.0.

11. The method according to any one of claims 8 to 10, wherein 75% or more of the leucine-rich α2 glycoprotein contained in the solvent is maintained without being decomposed after 29 days at 37 °C, optionally wherein 96% or more is maintained without being decomposed after 29 days at 37 °C.

## Patentansprüche

1. Leucinreiche α2-Glykoprotein-Zusammensetzung, umfassend leucinreiches a2-Glykoprotein, wobei der pH-Wert der Zusammensetzung 7,8 bis 9,0 beträgt, wobei die Konzentration eines Salzes der Zusammensetzung 300 mM bis 600 mM beträgt, wobei das Salz ein Natriumsalz, Kaliumsalz oder Magnesiumsalz ist und wobei die Konzentration des leucinreichen a2-Glykoproteins in der Zusammensetzung 0,1 bis 1000 µg/ml beträgt.

2. Leucinreiche α2-Glykoprotein-Zusammensetzung nach Anspruch 1, wobei das Salz ein Natriumsalz ist, wobei gegebenenfalls das Salz Natriumchlorid ist.

3. Leucinreiche α2-Glykoprotein-Zusammensetzung nach Anspruch 1 oder 2, wobei der pH-Wert der Zusammensetzung 8,0 bis 9,0 beträgt.

4. Leucinreiche α2-Glykoprotein-Zusammensetzung nach irgendeinem der Ansprüche 1 bis 3, wobei die α2-Glykoprotein-Zusammensetzung in einen Konservierungsbehälter (preservation container) abgefüllt ist.

5. Verwendung der leucinreichen
α2-Glykoprotein-Zusammensetzung nach irgendeinem der Ansprüche 1 bis 4 als eine Kalibrierungsprobenlösung (calibration sample solution) zur Messung der Konzentration von leucinreichem a2-Glykoprotein.

6. Verfahren zur Messung der Konzentration von leucinreichem a2-Glykoprotein, umfassend: Verwenden der leucinreichen a2-Glykoprotein-Zusammensetzung nach irgendeinem der Ansprüche 1 bis 4 als eine Kalibrierungsprobenlösung.

7. Kit, das die leucinreiche
α2-Glykoprotein-Zusammensetzung nach irgendeinem der Ansprüche 1 bis 4 umfasst.

8. Konservierungsstabilisierungsverfahren (preservation stabilizing method) für leucinreiches a2-Glykoprotein, umfassend:
Inkontaktbringen des leucinreichen a2-Glykoproteins mit einem Lösungsmittel mit einem pH-Wert von 7,8 bis 9,0,
wobei die Konzentration eines Salzes in dem Lösungsmittel 300 mM bis 600 mM beträgt, wobei das Salz ein Natriumsalz, Kaliumsalz oder Magnesiumsalz ist und
wobei die Konzentration des leucinreichen α2-Glykoproteins 0,1 bis 1000 µg/ml beträgt.

9. Konservierungsstabilisierungsverfahren für leucinreiches α2-Glykoprotein nach Anspruch 8, wobei das Salz ein Natriumsalz ist, wobei gegebenenfalls das Salz Natriumchlorid ist.

10. Konservierungsstabilisierungsverfahren für leucinreiches α2-Glykoprotein nach Anspruch 8 oder 9, wobei der pH-Wert der Zusammensetzung 8,0 bis 9,0 beträgt.

11. Verfahren nach irgendeinem der Ansprüche 8 bis 10, wobei 75% oder mehr des in dem Lösungsmittel enthaltenen leucinreichen a2-Glykoproteins erhalten bleiben, ohne nach 29 Tagen bei 37 °C zersetzt zu sein, wobei gegebenenfalls 96% oder mehr erhalten bleiben, ohne nach 29 Tagen bei 37 °C zersetzt zu sein.

## Revendications

1. Composition de glycoprotéine α2 riche en leucine comprenant une glycoprotéine α2 riche en leucine, dans laquelle un pH de la composition est de 7,8 à 9,0,
dans laquelle une concentration en sel de la composition est de 300 mM à 600 mM,
dans laquelle le sel est un sel de sodium, un sel de potassium ou un sel de magnésium, et
dans laquelle la concentration de la glycoprotéine α2 riche en leucine dans la composition est de 0,1 à 1 000 µg/mL.

2. Composition de glycoprotéine α2 riche en leucine selon la revendication 1, dans laquelle le sel est un sel de sodium, facultativement dans laquelle le sel est du chlorure de sodium.

3. Composition de glycoprotéine α2 riche en leucine selon la revendication 1 ou la revendication 2, dans laquelle la composition présente un pH de 8,0 à 9,0.

4. Composition de glycoprotéine α2 riche en leucine selon l'une quelconque des revendications 1 à 3, dans laquelle la composition de glycoprotéine α2 est conditionnée dans un récipient de conservation.

5. Utilisation de la composition de glycoprotéine α2 riche en leucine selon l'une quelconque des revendications 1 à 4 en tant que solution d'échantillon d'étalonnage pour mesurer la concentration en glycoprotéine α2 riche en leucine.

6. Procédé de mesure de la concentration en glycoprotéine α2 riche en leucine comprenant : l'utilisation de la composition de glycoprotéine α2 riche en leucine selon l'une quelconque des revendications 1 à 4 comme solution d'échantillon d'étalonnage.

7. Kit comprenant la composition de glycoprotéine α2 riche en leucine selon l'une quelconque des revendications 1 à 4.

8. Procédé de stabilisation de conservation pour la glycoprotéine α2 riche en leucine comprenant :
la mise en contact de la glycoprotéine α2 riche en leucine avec un solvant présentant un pH de 7,8 à 9,0, dans lequel la concentration en sel du solvant est de 300 mM à 600 mM,
dans lequel le sel est un sel de sodium, un sel de potassium ou un sel de magnésium, et
dans lequel la concentration de la glycoprotéine α2 riche en leucine est de 0,1 à 1 000 µg/mL.

9. Procédé de stabilisation de conservation pour la glycoprotéine α2 riche en leucine selon la revendication 8, dans lequel le sel est un sel de sodium, facultativement dans lequel le sel est du chlorure de sodium.

10. Procédé de stabilisation de conservation de la glycoprotéine α2 riche en leucine selon la revendication 8 ou la revendication 9, dans lequel la composition présente un pH de 8,0 à 9,0.

11. Procédé selon l'une quelconque des revendications 8 à 10, dans lequel 75 % ou plus de la glycoprotéine α2 riche en leucine contenue dans le solvant est maintenu sans dégradation après 29 jours à 37 °C, facultativement, dans lequel 96 % ou plus est maintenu sans dégradation après 29 jours à 37 °C.
